# EUROPÄISCHE PATENTSCHRIFT

(11) **EP 0 948 266 B1**
(45) Veröffentlichungstag und Bekanntmachung des Hinweises auf die Patenterteilung: **02.05.2003**
(21) Anmeldenummer: 97951900.6
(22) Anmeldetag: 14.11.1997
(51) Int. Cl.: A23L 2/52, A23L 2/54

(54) **UBICHINON ENTHALTENDES NICHT ALKOHOLISCHES GETRÄNK**
UBIQUINONE-CONTAINING, NON-ALCOHOLIC BEVERAGE
BOISSON NON ALCOOLIQUE CONTENANT DE L'UBIQUINONE

(30) Priorität: 15.11.1996 DE 19647352
(43) Veröffentlichungstag der Anmeldung: 13.10.1999
(73) Patentinhaber: Aqua Nova Getränketechnologie GmbH, 68219 Mannheim (DE)
(72) Erfinder: BEHNAM, Dariush, D-64380 Ro dorf (DE); ELSTNER, Erich, F., D-82194 Gröbenzell (DE); WANNENMACHER, Michael, D-69118 Heidelberg (DE)
(74) Vertreter: Zinngrebe, Horst, Dr.rer.nat.
(86) Internationale Anmeldenummer: EP9706360
(87) Internationale Veröffentlichungsnummer: WO98021984

(56) Entgegenhaltungen:
- EP-A- 0 325 244
- WO-A-88/06411
- WO-A-95/05093
- US-A- 3 499 088
- US-A- 4 824 669
- US-A- 5 290 605
- DATABASE WPI Section Ch, Week 9608 Derwent Publications Ltd., London, GB; Class B05, AN 96-074749 XP002059918 & JP 07 330 593 A (TAISHO PHARM CO LTD) , 19.Dezember 1995
- DATABASE WPI Section Ch, Week 9523 Derwent Publications Ltd., London, GB; Class B04, AN 95-174960 XP002059919 & JP 07 096 282 A (NIPPONDENSO CO LTD) , 11.April 1995

## Beschreibung

Der mitochondriale Elektronentransport der Atmungskette setzt sich aus verschiedenen Komponenten zusammen, von denen eine das Coenzym Q (Ubichinon) darstellt. Das Ubichinon hat eine interessante Funktion deshalb, weil es zwischen einem 2-Elektronentransport, vom Elektronendonator NADH oder Succinat herkommend in einen 1-Elektronentransport der Cytochromkette überleitet. Diese Funktion des Ubichinons ist seit langer Zeit bekannt. Die Ubichinonstelle im mitochondrialen Elektronentransport ist eine Schaltstelle. Von verschiedenen Donatoren kommen Elektronen auf das Ubichinon zu, reduzieren es zum Hydrochinon, welches seinerseits wieder Elektronen auf die Cytochromkette abgibt. Dabei wechselen sich 1- und 2-Elektronenübergänge ab, so daß ein Hydrochinon-Semichinon-Chinonzyklus überlagert ist. Durch diesen Elektronenübergang werden nicht nur Reduktionsäquivalente, sondern auch Protonen über die Mitochondrienmembran geschleust, was zu einem Protonengradienten führt, welcher wiederum für die ATP-Synthese ausgenutzt wird.

Somit ist Ubichinon ganz wesentlich an der Umwandlung der in der Nahrung enthaltenen Energie in körpereigene Energie (ATP) beteiligt. CoQ10 wird daher oft als Energieaktivator bezeichnet.

Bei diversen sportmedizinischen Untersuchungen unter Einsatz von CoQ10 bei Leistungssportlern konnte gezeigt werden, daß eine Korrelation zwischen Leistungssteigerung und dem erhöhten CoQ10-Plasmaspiegel besteht.

Ursachen einer unzureichenden CoQ10-Versorgung sind:

### a) Reduzierte Q10-Synthese

Es ist bekannt, daß mit zunehmenden Lebensalter die Q10-Konzentration in Geweben verschiedener Organe, insbesondere im Herz, stark abnimmt.

Der Abfall der CoQ10-Spiegel ist höher als der altersbedingte Abfall anderer Lipide im menschlichen Organismus.

### b) Erhöhter Bedarf an CoQ10:

Bei hoher körperlicher Belastung wie z. B. Sport, Schwerarbeit oder bei Streß benötigt der Körper entsprechend größere Mengen an CoQ10. Grund hierfür ist der erhöhte Energieumsatz, der das Elektronentransportsystem stärker beansprucht, wodurch Ubichinon CoQ10 in erhöhtem Umfang benötigt wird.
Bei Belastungssituationen wie beim Sport ist die CoQ10-Spiegel im Plasma vermindert.

### c) Reduzierte alimentäre Aufnahme:

CoQ10 ist zwar in verschiedenen Nahrungsmitteln wie Fisch, Fleisch, Sojabohnen, Mais und Nüssen vertreten, aber seine Temperaturempfindlichkeit bedingt, daß es beim Kochen oder Rösten oft fast vollständig zerstört wird.

Bei verschiedenen Krankheitsbildern, ist ein starker Ubichinonabfall zu messen. Dieser Abfall betrifft aber nicht nur das Ubichinon, welches man in den Mitochondrien findet, sondern auch vor allem das Ubichinon, welches im Cytoplasma oder an andere Organellen gebunden vorliegt. Auf der anderen Seite gibt es aber Hinweise dafür, daß sportliches Training (physical exercise) nicht nur die üblichen Antioxidantien hochfährt, sondern auch die Ubichinonsynthese stimuliert. Diese Tatsache deutet bereits auf eine Funktion von Ubichinon hin, die zusätzlich zu der eines Elektronentransportmetaboliten im intermediären Metabolismus stattfindet: der einer antioxidativen Funktion im Sinne der Protektion vor reaktiven Sauerstoffspezies.

Wie erwähnt, findet man Ubichinon nicht nur in der mitochondrialen Respirationskette, sondern in allen zellulären Membranen und im Blutserum und auch in den Serumlipoproteinen (vor allem im LDL.) (Ernster und Dallner, 1995). So ist es nicht verwunderlich, daß Kontush et al fanden, daß Ubichino Q 10 ein effektiver Schutzfaktor gegen die Oxidation von LDL durch Kupferionen fungiert (Kontush et al. 1995). Wie mehrere Autoren berichteten, ist es vor allem das reduzierte Coenzym Q, welches diese protektive Funktion ausübt. So ist es nicht verwunderlich, daß Coenzym Q durch membrangebundene NADPH- oder NADH-Reduktasen (DT-Diaphorasen) reduziert wird (Takahashi et al. 1995; Beyer et al. 1996). In der reduzierten Form schützt Coenzym Q vor allem mitochondriale Membranen gegen von Adriamyzin (ein Chemotherapeutikum) ausgelöste Oxidationsvorgänge (Beyer et al. 1996). Bei dieser Schutzfunktion scheint das Coenzym Q kooperativ zu anderen Antioxidantien zu wirken, vor allem mit dem Vitamin E und der Selenoperoxidase (Glutationperoxidase) (Chen und Tappel 1994). Somit scheint das Coenzym Q und das α-Tocopherol kooperativ in der gesamten Hackordnung der Antioxidantien zu fungieren (Noack et al. 1994, Beyer 1994). Auch eine Interaktion mit Haemproteinen ist gezeigt worden, wobei eine Recyclisierung des Ferrylstatus von Haemproteinen durch Coenzym Q einen neuartigen antioxidativen Mechanismus darstellen könnte (Mordente et al. 1994).

Darüber hinaus wurde für Q 10 auch ein immunmodulatorischer Effekt derart angenommen, daß ein Defizit an Q 10 im Myokard zu entzündlichen Reaktionen führt, welche durch Q 10-Gaben verbessert werden können (Folkers und Wolaniuk 1985).
Effekte auf das ischämische Herz oder auf überbeanspruchtes Myokard wurden für Coenzym Q am häufigsten beschrieben, wobei eine Stimulierung der kardialen Funktion beobachtet wurde (Hano et al. 1994, Chrestanello et al. 1996; Morita et al. 1995).

Wie aus zahlreichen Publikationen ersichtlich, sind die Effekte auf die myokardiale Energiezufuhr durch Ubichinon 10-Gaben am besten dokumentiert und eine Therapie mit 100 mg täglich wurde als sehr hilfreich bei Therapien diverser Herzerkrankungen angesehen (Mortensen 1993). Auch andere Organe, wie z.B. perfundierte Rattenleber (Genova et al. 1994) oder exzessiver Alkoholmetabolismus (Loop et al. 1994), Stabilität präservierter Lungen (Hanagiri et al. 1994), periodontale Probleme im Zusammenhang mit mangelnder Hygiene (Wilkinson et al. 1976) Bypassoperationen (Morita 1995) sowie supplementäre Funktionen bei der Regulation des Homocystein-Methionin-Stoffwechsls im Zusammenhang mit defizitärer Lage der Vitamine B6, B12 und Folsäure (Sinatra und DeMarco 1995) wurden neben der Schutzfunktion des LDLs als äußerst bedeutsam beschrieben.

US-A-3 499 088 offenbart ein Verfahren zur Herstellung einer stabilen wäßrigen Lösung von Koenzym Q10, wobei Koenzym Q10 mit Polyoxyethylen-hydrogeniertes Kastoröl und Propylenglykol vermischt wird und dann Wasser zugesetzt wird

Der Erfindung liegt die Aufgabe zugrunde, ein gut verträgliches, leicht applizierbares Mittel zu entwickeln, das es erlaubt, dem Organismus gleichsam beiläufig eine wünschenswerte Dosis an Ubichinon Q10 zuzuführen.

Dazu dient ein nicht alkoholisches Getränk, wie Tafelwasser, Heilwasser oder dergleichen, das 10 mg/l bis 500 mg/l des Ubichinon Q 10 enthält. Da der übliche Konsum von beispielsweise Tafelwasser allgemein verbreitet und relativ hoch ist, wird durch die Zugabe von Q 10 zu einem derartigen Tafelwasser dem Organismus gleichzeitig das Q 10 zugeführt.

Insbesondere dann, wenn das Ubichinon Q 10 als Lösung zugesetzt ist, wobei als Lösungsvermittler vorteilfafterweise Polyoxyethylen-Sorbitanmonooleat (Polysorbat 80, E 433) benutzt wird, liegen Q 10-haltige Getränke in klarer, trübungsfreier Form vor. Nach der Herstellung wird das Getränk in lichtundurchlässige Behälter abgefüllt, die insbesondere für das nahe und ferne ultraviolette Licht undurchlässig sind. Hierbei können auch Behälter, beispielsweise Dosen, verwendet werden, die das gesamte Lichtspektrum abdecken. Bevorzugterweise werden Dosen aus Aluminium oder Aluminiumlegierungen verwendet. Auch kann das erfindungsgemäße Getränk in Metallfolienoder Alufolien-Beutel sowie in dem an sich bekannten Tetrapak aufgenommen werden. Ferner kommen bestimmte Kunststoffe oder Naturmaterialien wie Stein und Ton in Frage. Wenn das erwähnte Material selbst nicht die gewünschte Lichtundurchlässigkeitseigenschaft aufweist, kann es beschichtet werden. Auch besteht die Möglichkeit eine lichtundurchlässige Umverpackung zu verwenden.

Ein Verfahren zur Herstellung eines nicht alkoholischen Getränkes wie Tafelwasser, Heilwasser oder dergleichen zeichnet sich nach der Erfindung dadurch aus, daß das Ubichinon Q 10 in einem Lösungsvermittler aufgelöst und die Lösung dem Getränk zugesetzt wird. Als Lösungsvermittler kommt bevorzugt Polyoxyethylen-Sorbitanmonooleat (Polysorbat 80, E433) in Betracht.

Die Wirksamkeit des Getränks wird erweitert, wenn es über seinen natürlichen Sauerstoffgehalt hinaus mit gelöstem Sauerstoff angereichert wird, so daß es zweckmäßig einen Sauerstoffgehalt von 10 mg/l bis 85 mg/l an gelöstem Sauerstoff enthält. Bei plötzlicher Sauerstoffzufuhr zu isolierten Rinderherz-Mitochondrien, welche unter hypoxischen Bedingungen inkubiert waren, ergeben sich sowohl in den Mitochondrien als auch in submitochondrialen Partikeln Lipidperoxidationen sowie eine oxidative Schädigung bestimmter Proteine. Diese Schädigung kann durch Ubichinon gemildert werden.

Auf der anderen Seite konnte gezeigt werden (M.J. Eble, F. Lohr, M. Wannenmacher in ONKOLOGIE, April 1995, Seite 136, sowie von M.J. Eble, B. Vanselow, A. Dietz , M. Wannenmacher in einer Untersuchung, über die auf dem 2. deutschen Kongreß für Radioonkologie, Strahlenbiologie und medizinische Physik in Baden-Baden vom 16. bis 19. November 1996 berichtet worden ist) daß der erhöhte Sauerstoffgehalt in einem Getränk, insbesondere Wasser über einen noch nicht bekannten Bahnungseffekt die Durchblutung hypoxischer Gewebebereich sehr stark fördert. Bei dieser Förderung kann es, wie auch bei sportlicher Betätigung, kurzfristig zu einem Absinken des antioxidativen Potentials kommen, wie für zahlreiche ischämische und Perfusionsmodelle gezeigt wurde. Eine prophylaktische Beimengung von Q 10 und Vitamin E zu dem Getränk annuliert diese Effekte bereits im Vorfeld.

Mit besonderem Vorteil wird dem Getränk Selen in einer vom Organismus verwertbaren Form zugegeben, beispielsweise in einer Selenkonzentration von 5 µg/l bis 500 µg/l. Hierzu wurden auf dem 6. Internationalen Selen-Symposium in Peking die Ergebnisse einer prospektiven randomisierten U.S. amerikanischen Doppelblindstudie mit 1312 Patienten vorgestellt, die an nicht melanomatösen Hautkrebserkrankungen litten. Dabei zeigte sich, daß die tägliche Gabe von 200 µg Selen die Krebsterblichkeit um 25 %, die sekundäre Krebsinzidenz um 52 %, das Lungenkrebsrisiko um 40 %, die Lungenkrebsmortalität um 49 %, die kolorektale Karzinominzidenz um 64 %, die Prostatakarzinominzidenz um 59 % und die Bronchialkarzinominzidenz um 40 % sanken.

Der gesamte Herstellungs- und Abfüllprozeß erfolgt zweckmäßig unter Lichcausschluß.

In einem Ausführungsbeispiel des erfindungsgemäßen Getränks wird zunächst von dem Wasser der Renata-Quelle in D-64757 Rothenberg ausgegangen. Dessen am 24. Mai 1995 durchgeführte Analyse ergab Werte, die in der deutschen Patenschrift 195 29 955 Spalten 3 und 4 angegeben sind. Auf diese Werte wird hier ausdrücklich Bezug genommen.

30 mg Ubichinon Q 10 wurden in 105 mg auf ca. 60° erwärmtem Polyoxyethylen-Sorbitanmonooleat (erhältlich unter dem Handelsnamen Lamesorb SMO 20 von der Grünau GmbH in D-89251 Illertissen, Postfach 10 63) zu einer klaren, etwas gelblichen Lösung aufgelöst. Diese Lösung wurde 0,33 1 des vorstehend erwähnten Quellwassers zugegeben und oxygeniert. Die Oxygenierung geschieht in einer Weise, wie das in der internationalen Patentanmeldung mit der Veröffentlichungsnummer WO95/32796 im einzelnen beschrieben ist. Nach der Anreicherung mit Sauerstoff in der dort beschriebenen Weise ergab sich ein Gehalt von Sauerstoff (O₂) von etwa 79 mg/l. Das ergab ein klares Getränk.

In einem weiteren Schritt wurde dem Getränk der Inhalt einer 10 ml Injektionsflasche zugegeben, die 500 µg Selen enthielt (erhältlich unter dem Handelsnamen "selenase" von der G.N. FARM Arzneimittel GmbH, Schorndorferstr. 32, D-70734 Fellbach). Nach der Zugabe wurde ein leicht trinkbares Getränk erhalten, das seinen Quellwasser-Charakter beibehalten hatte.

Wenn gewünscht können dem Getränk ein oder mehrere Frucht- und/oder Gemüsesaftkonzentrate und/oder Geschmacksverbesserer zugegeben werden.

Ergänzend wird das Getränk mit B₁ und/oder B₂ und/oder B₃ und/oder Vitamin E vitaminisiert. Vitamin E beseitigt mögliche Schieflagen des antioxidativen Potentials mit Q 10. Die Zugabe von Vitamin E zum Getränk erfolgt als Lösung, wobei für diese Lösung der bereits für das Ubichinon Q 10 verwendete Lösungsvermittler eingesetzt wird. Das erfindungsgemäße Getränk wirkt sich therapieunterstützend sowohl bei neurodegenerativen Störungen, depressiven Auslenkungen wie auch bei atherogenen Prozessen (Alter, Rauchen) aus.

Um den Geschmack des als Nahrungsergänzungsmittel benutzbaren Getränkes zu verbessern, kann es aromatisiert werden. Dazu empfiehlt sich eine Mischung aus 1,38 g/l Limette, 1,04 g/l Cassis und 1,04 g/l Mango. Weiterhin ist zu empfehlen, etwa 20 g/l Maltodextrin und etwa 50 g/l Fructose zuzusetzen. Das fertige Getränk wird zweckmäßig einer Vorund gegebenenfalls einer Nachfiltration unterworfen, wobei sich Filter mit einer Porengröße von 0,1 µ bis 1,5 µ bewährt haben. Danach kann das Getränk in lichtundurchlässige, mindestens aber im Ultravioletten undurchlässige Behälter wie etwa Dosen abgefüllt werden. In diesen besitzt das klare Getränk eine ausgezeichnete Lagerfähigkeit.

## Patentansprüche

1. Klares, nicht alkoholisches Getränk wie Tafelwasser, Heilwasser oder dergleichen, **gekennzeichnet durch** einen Gehalt an 10 mg/l bis 500 mg/l Ubichinon Q₁₀ sowie einen Lösungsvermittler, bevorzugt Polysorbat 80.

2. Getränk nach Anspruch 1, **gekennzeichnet durch** einen Gehalt an Selen in vom Organismus verwertbarer Form.

3. Getränk nach Anspruch 2, **dadurch gekennzeichnet, dass** die Selenkonzentration 5µg/l bis 500 µg/l beträgt.

4. Getränk nach einem der vorstehenden Ansprüche, **dadurch gekennzeichnet, dass** ein oder mehrere Fruchtsaftkonzentrate und/oder Geschmacksverbesserer zugesetzt sind.

5. Getränk nach einem der vorstehenden Ansprüche, **dadurch gekennzeichnet, dass** das Ubichinon als Lösung zugesetzt ist.

6. Getränk nach einem der vorstehenden Ansprüche **gekennzeichnet durch** einen Gehalt an Vitamin E.

7. Getränk nach Anspruch 6, **dadurch gekennzeichnet, dass** das Vitamin E als Lösung zugesetzt ist.

8. Getränk nach einem der vorstehenden Ansprüche, **dadurch gekennzeichnet, dass** es in einem lichtundurchlässigen Behälter aufgenommen ist.

9. Getränk nach Anspruch 8, **dadurch gekennzeichnet, dass** der Behälter für das nahe und das ferne ultraviolette Licht undurchlässig ist.

10. Getränk nach einem der vorstehenden Ansprüche, **dadurch gekennzeichnet, dass** es aromatisiert ist.

11. Getränk nach Anspruch 10, **dadurch gekennzeichnet, dass** es die Limette von 1,38 g/l, Cassis von 1,04 g/l und Mango von 1,04 g/l enthält.

12. Getränk nach einem der vorstehenden Ansprüche **gekennzeichnet durch** einen Zusatz von 20 g/l Maltodextrin und 50 g/l Fructose.

13. Zwischenprodukt zur Herstellung eines Getränks nach einem der vorstehenden Ansprüche, **dadurch gekennzeichnet, dass** 30 mg Ubichinon Q₁₀ in 105 mg auf circa 60° erwärmtem Polysorbat 80 zu einer klaren Lösung aufgelöst sind.

14. Verfahren zur Herstellung eines nicht-alkoholischen Getränks nach einem der vorstehenden Ansprüche, **dadurch gekennzeichnet, dass** Ubichinon Q₁₀ in einem Lösungsvermittler aufgelöst und die Lösung dem Getränk zugesetzt wird.

15. Verfahren nach Anspruch 14, **dadurch gekennzeichnet, dass** als Lösungsvermittler bevorzugt Polysorbat 80 eingesetzt wird.

16. Verfahren nach Anspruch 15, **dadurch gekennzeichnet, dass** das Polysorbat 80 auf circa 60° erwärmt wird.

17. Verfahren nach einem der Ansprüche 14 bis 16, **dadurch gekennzeichnet, dass** dem Getränk Selen in vom Organismus verwertbarer Form und/oder Vitamin E zugesetzt wird.

18. Verfahren nach Anspruch 17, **dadurch gekennzeichnet, dass** das Vitamin E vor dem Zusetzen in Polysorbat 80 aufgelöst wird.

## Claims

1. Nonalcoholic clear beverage Comprising from 10 to 500 mg/l of ubiquinone Q10 and a solubilizer, preferably polysorbate 80.

2. Beverage according to claim 1, further comprising selenium in a form which can be utilized by the body.

3. Beverage according to claim 2, wherein the selenium concentration is 5 to 500 µg/l.

4. Beverage according to one of the previous claims wherein one or more fruit juice concentrates and/or flavor improvers are added to the beverage.

5. Beverage according to one of the previous claims wherein ubiquinone Q10 as a solution is added to the beverage.

6. Beverage according to one of the previous claims further comprising vitamin E.

7. Beverage according to claim 6, wherein vitamin E as a solution is added to the beverage.

8. Beverage according to one of the previous claims wherein the beverage is in an opaque container.

9. Beverage according to claim 8, wherein the container is opaque to near and far ultraviolet light.

10. Beverage according to one of the previous claims wherein the beverage is aromatized.

11. Beverage according to claim 10, wherein the beverage contains 1.38 g/l of lime, 1.04 g/l of cassis, and 1.04 g/l of mango.

12. Beverage according to one of the previous claims wherein the beverage contains additionally 20 g/l maltodextrin and 50 g/l fructose.

13. Interim product for the production of a beverage according to one of the previous claims wherein 30 mg ubiquinone Q10 are dissolved to a clear solution in 105 mg polysorbate 80 heated to approximately 60°C.

14. Process for the production of a nonalcoholic beverage according to one of the previous claims, dissolving ubiquinone Q10 in a solubilizer and adding the solution to the beverage.

15. Process according to claim 14, wherein the primarily used solubilizer is polysorbate 80.

16. Process according to claim 15, wherein the polysorbate 80 is heated to approximately 60°C.

17. Process according to one of the claims 14, to 16, further comprising adding vitamin E and/or selenium to the beverage in a form which can be utilized by the body.

18. Process according to claim 17, wherein the vitamin E is dissolved in polysorbate 80 before it is added to the beverage.

## Revendications

1. Boisson claire non alcoolique telle qu'eau potable, eau minérale ou similaire, **caractérisée par** une teneur en ubiquinone Q₁₀ de 10 mg/l à 500 mg/l de même qu'en un agent solubilisant, préférentiellement le polysorbate 80.

2. Boisson suivant la revendication 1, **caractérisée par** une teneur en sélénium sous une forme utilisable par l'organisme.

3. Boisson suivant la revendication 2, **caractérisée en ce que** la concentration en sélénium va de 5 µg/l à 500 µg/l.

4. Boisson suivant l'une des revendications précédentes, **caractérisée en ce qu'**elle est additionnée d'un ou plusieurs concentrés de jus de fruits et/ou d'une ou plusieurs substances améliorant le goût.

5. Boisson suivant l'une des revendications précédentes, **caractérisée en ce que** l'ubiquinone est ajoutée sous forme de solution.

6. Boisson suivant l'une des revendications précédentes, **caractérisée par** une teneur en vitamine E.

7. Boisson suivant la revendication 6, **caractérisée en ce que** la vitamine E est ajoutée sous forme de solution.

8. Boisson suivant l'une des revendications précédentes, **caractérisée en ce qu'**elle est reçue dans un récipient opaque à la lumière.

9. Boisson suivant la revendication 8, **caractérisée en ce que** le récipient est opaque à la lumière dans le proche ultraviolet et dans l'ultraviolet lointain.

10. Boisson suivant l'une des revendications précédentes, **caractérisée en ce qu'**elle est aromatisée.

11. Boisson suivant la revendication 10, **caractérisée en ce qu'**elle contient 1,38 g/l de limette, 1,04 g/l de cassis et 1,04 g/l de mangue.

12. Boisson suivant l'une des revendications précédentes, **caractérisée par** une addition de 20 g/l de maltodextrine et de 50 g/l de fructose.

13. Produit intermédiaire pour la préparation d'une boisson selon l'une des revendications précédentes, **caractérisé en ce que** 30 mg d'ubiquinone Q₁₀ sont dissous dans 105 mg de polysorbate 80 chauffé à environ 60° en formant une solution limpide.

14. Procédé de production d'une boisson non alcoolique suivant l'une des revendications précédentes, **caractérisé en ce que** de l'ubiquinone Q₁₀ est dissoute dans un agent solubilisant et la solution est ajoutée à la boisson.

15. Procédé suivant la revendication 14, **caractérisé en ce que** du polysorbate 80 est utilisé préférentiellement comme agent solubilisant.

16. Procédé suivant la revendication 15, **caractérisé en ce que** le polysorbate 80 est chauffé à environ 60°.

17. Procédé suivant l'une des revendications 14 à 16, **caractérisé en ce que** du sélénium sous une forme utilisable dans l'organisme et/ou de la vitamine E sont ajoutés à la boisson.

18. Procédé suivant la revendication 17, **caractérisé en ce que** la vitamine E est dissoute dans du polysorbate 80 avant son addition.
